# EUROPEAN PATENT APPLICATION

(11) **EP 4 342 897 A1**
(43) Date of publication of application: **27.03.2024**
(21) Application number: 22804072.1
(22) Date of filing: 20.05.2022
(51) Int. Cl.: C07D 471/04, C07D 519/00, A61K 31/5377, A61P 35/00

(54) **PHARMACEUTICALLY ACCEPTABLE SALT OF PYRAZOLOHETEROARYL DERIVATIVE AND CRYSTAL FORM THEREOF**

(30) Priority: 21.05.2021 CN 202110558670
(71) Applicant: Jiangsu Hengrui Pharmaceuticals Co., Ltd., Lianyungang, Jiangsu 222047 (CN); Shanghai Hengrui Pharmaceutical Co. Ltd., Minhang District Shanghai 200245 (CN)
(72) Inventor: YAO, Jiaqi, Lianyungang, Jiangsu 222047 (CN); YANG, Junran, Lianyungang, Jiangsu 222047 (CN); DU, Zhenxing, Lianyungang, Jiangsu 222047 (CN)
(74) Representative: dompatent von Kreisler Selting Werner - Partnerschaft von Patent- und Rechtsanwälten mbB
(86) International application number: PCT/CN2022/094161
(87) International publication number: WO 2022/242753

(57) **Abstract**

The present disclosure relates to a pharmaceutically acceptable salt of a pyrazoloheteroaryl derivative and a crystal form thereof. In particular, the present disclosure relates to a pharmaceutically acceptable salt of a compound represented by formula (I), and a crystal form thereof. The novel crystal form of the present disclosure has good physical and chemical properties.

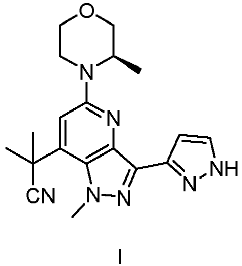

## Description

The present application claims priority to the Chinese Patent Application No. 2021105586705 filed on May 21, 2021, which is incorporated herein by reference in its entirety.

### TECHNICAL FIELD

The present disclosure relates to a pharmaceutically acceptable salt of a pyrazolo-heteroaryl derivative and a crystal form thereof, and particularly to a pharmaceutically acceptable salt of a compound of formula (I) and a crystal form thereof.

### BACKGROUND

Thousands of DNA damages occur every day in both normal and tumor cells. This makes DNA damage repair a crucial role in maintaining genomic stability and cell viability. Compared to normal cells, tumor cells are subject to greater replication stress. They carry more endogenous DNA damages, and often demonstrate loss of one or more DNA damage repair pathways. This makes the survival of tumor cells more dependent on the successful repair of DNA damages. Homologous recombination repair is the prominent repair mode of DNA double-strand break, which takes the homologous sequence of undamaged sister chromatid as the template for repair to replicate the DNA sequence at the damaged part and precisely repair the DNA. This repair occurs primarily in the G2 and S phases. ATR, a member of the PIKK family, is a key enzyme in the homologous recombination repair pathway. When ATR/ATRIP complex binds to damaged DNA covered by replication protein A (RPA), ATR is activated and regulates checkpoints of the cell cycle by phosphorylating downstream proteins such as Chk1 and SMARCAL, causing cell cycle arrest. It also ensures the stability of damaged DNA and elevates dNTP concentration to promote DNA damage repair. DNA damage repair occurring during the S phase of the cell cycle is mainly accomplished by the ATR pathway, suggesting that ATR is very important to ensure cell proliferation. Analysis of clinical tumor samples indicated that elevated ATR expression levels were observed in a variety of tumor tissues, such as gastric cancer, liver cancer, colorectal cancer, ovarian cancer, and pancreatic cancer. Moreover, in patients with ovarian cancer and pancreatic cancer, a higher level of ATR is usually associated with a lower survival rate. As such, ATR is an important target for tumor therapy.

WO2021098811A relates to a series of novel ATR inhibitors, wherein the compound of formula (I) has good ATR inhibitory activity and its structure is shown below:

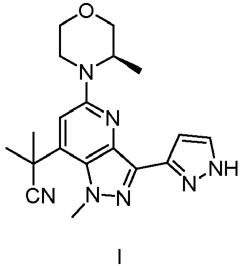

The structures of crystalline forms of a pharmaceutical active ingredient and intermediates thereof generally affect their chemical stability, and the differences in crystallization conditions and storage conditions may cause changes in the structure of the crystalline form of the compound and sometimes generation of other crystalline forms. Generally, amorphous products have no regular crystalline form structure and often have other defects, such as poor product stability, fine powder, difficulty in filtration, ease of agglomeration and poor flowability. Therefore, it is necessary to improve various properties of the above products, and intensive research is needed to explore novel crystalline forms with high purity and chemical stability.

### SUMMARY

The present disclosure provides a novel salt form of a compound of formula (I), a crystalline form thereof and a method for preparing the same.

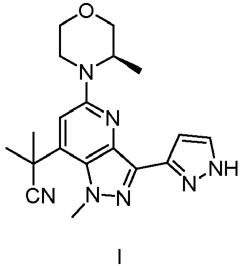

The present disclosure provides a pharmaceutically acceptable salt of a compound of formula (I), wherein the pharmaceutically acceptable salt is selected from the group consisting of hydrochloride, sulfate, hydrobromide, mesylate, p-toluenesulfonate, maleate, phosphate, formate, acetate, succinate, fumarate, citrate, malate, hippurate and oxalate.

In certain embodiments, the pharmaceutically acceptable salt is mesylate, maleate, or oxalate.

In certain embodiments, the molar ratio of the compound of formula (I) to sulfuric acid in the sulfate is 3:1 to 1:3, preferably 1:0.5 or 1:1.

In certain embodiments, the molar ratio of the compound of formula (I) to maleic acid in the maleate is 3:1 to 1:3, preferably 1:0.5 or 1:1.

In certain embodiments, the molar ratio of the compound of formula (I) to *p*-toluenesulfonic acid in the *p*-toluenesulfonate is 3:1 to 1:3, preferably 1:1 or 1:2.

In certain embodiments, the molar ratio of the compound of formula (I) to methanesulfonic acid in the mesylate salt is 3:1 to 1:3, preferably 1:1 or 1:2.

In certain embodiments, the molar ratio of the compound of formula (I) to oxalic acid in the oxalate is 3:1 to 1:3, preferably 1:0.5 or 1:1.

The present disclosure also provides a crystalline form of hydrochloride of a compound of formula (I), wherein the crystalline form is:
a crystalline form a of hydrochloride having an X-ray powder diffraction pattern with characteristic peaks at 2θ angles of 6.0, 8.3, 12.1, 14.3, 14.9, 16.7 and 26.7;
a crystalline form b of hydrochloride having an X-ray powder diffraction pattern with characteristic peaks at 2θ angles of 6.0, 12.1, 18.2, 23.6 and 24.4.

In certain embodiments, the crystalline form a of hydrochloride has an X-ray powder diffraction pattern with characteristic peaks at 2θ angles of 6.0, 8.3, 9.1, 12.1, 14.3, 14.9, 16.7, 18.3, 19.4, 23.5, 24.3, 26.3 and 26.7.

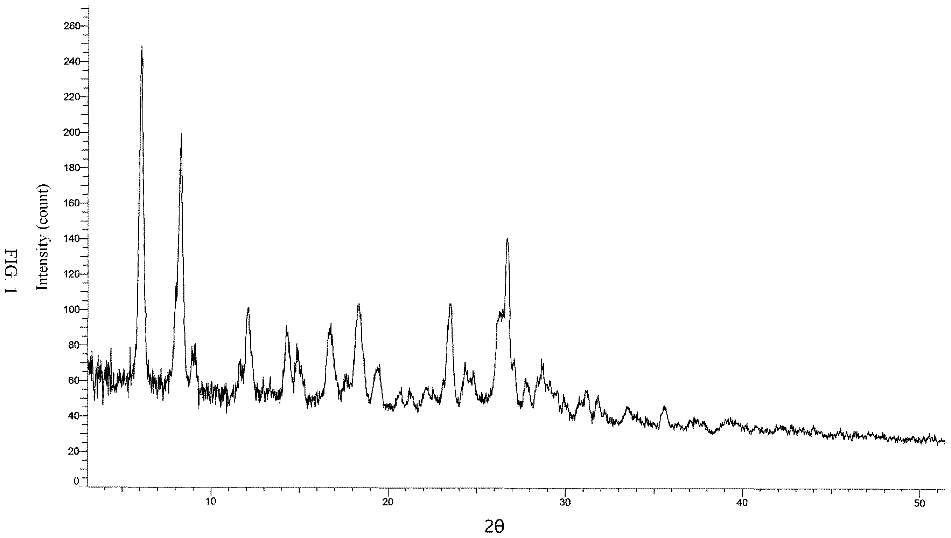

In certain embodiments, the crystalline form a of hydrochloride has an X-ray powder diffraction pattern shown in FIG. 1.

In certain embodiments, the crystalline form b of hydrochloride has an X-ray powder diffraction pattern with characteristic peaks at 2θ angles of 6.0, 8.4, 9.0, 12.1, 16.5, 18.2, 23.6, 24.4, 26.2, 29.5, 33.9 and 35.5.

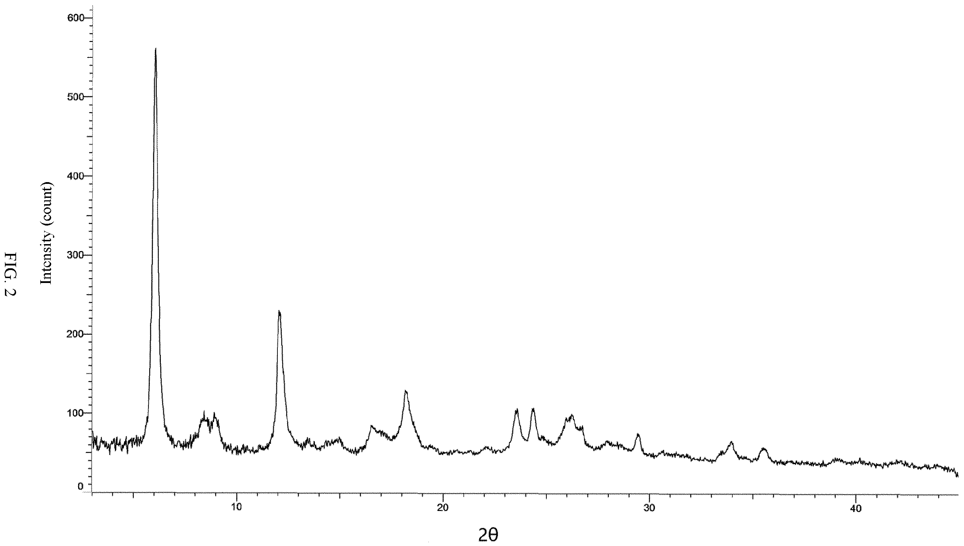

In certain embodiments, the crystalline form b of hydrochloride has an X-ray powder diffraction pattern shown in FIG. 2.

The present disclosure also provides a crystalline form α of sulfate of a compound of formula (I), which has an X-ray powder diffraction pattern with characteristic peaks at 2θ angles of 5.8, 7.6, 13.7, 15.4 and 20.4.

In certain embodiments, the crystalline form α of sulfate has an X-ray powder diffraction pattern with characteristic peaks at 2θ angles of 5.8, 7.6, 13.7, 15.4, 16.4, 16.9, 18.0, 18.5, 19.2, 20.4, 23.0, 23.9 and 25.9.

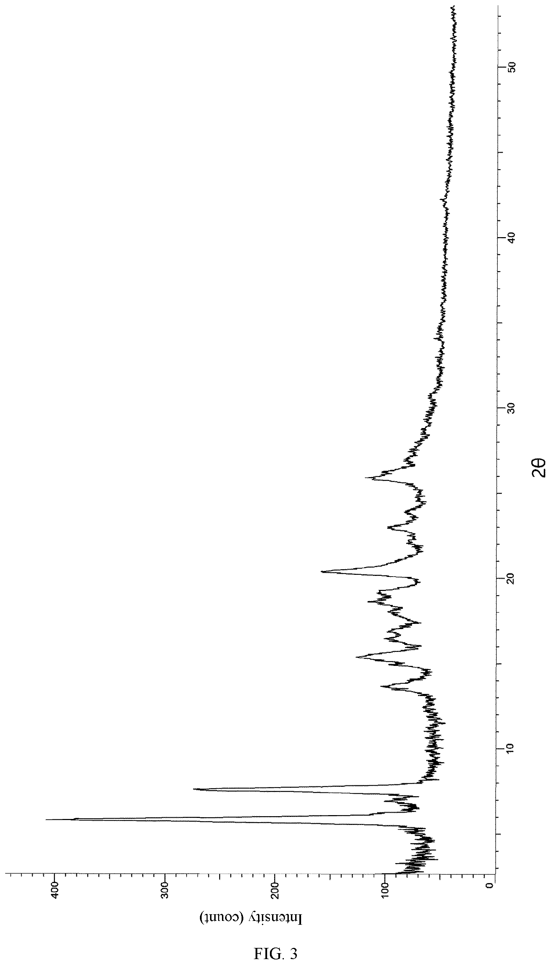

In certain embodiments, the crystalline form α of sulfate has an X-ray powder diffraction pattern shown in FIG. 3.

The present disclosure also provides a crystalline form of hydrobromide of a compound of formula (I), wherein the crystalline form is:
a crystalline form I of hydrobromide having an X-ray powder diffraction pattern with characteristic peaks at 2θ angles of 6.0, 8.1, 14.7, 25.9 and 27.0;
a crystalline form II of hydrobromide having an X-ray powder diffraction pattern with characteristic peaks at 2θ angles of 9.3, 11.6, 13.0, 16.8, 18.7 and 24.6.

In certain embodiments, the crystalline form I of hydrobromide has an X-ray powder diffraction pattern with characteristic peaks at 2θ angles of 6.0, 8.1, 14.7, 17.3, 18.8, 22.0, 25.9, 27.0 and 27.8.

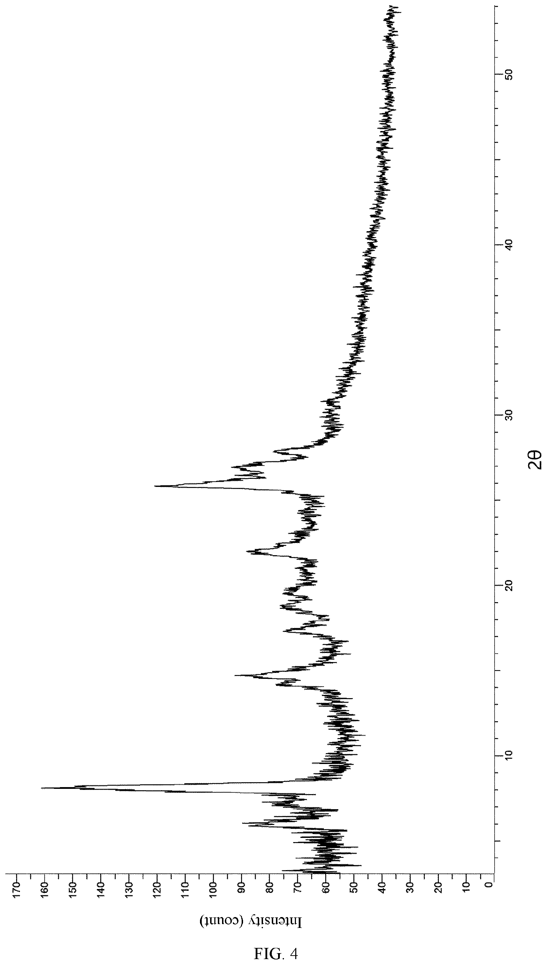

In certain embodiments, the crystalline form I of hydrobromide has an X-ray powder diffraction pattern shown in FIG. 4.

In certain embodiments, the crystalline form II of hydrobromide has an X-ray powder diffraction pattern with characteristic peaks at 2θ angles of 8.2, 9.3, 11.6, 13.0, 15.5, 16.8, 17.6, 18.7, 19.3, 19.8, 21.3, 22.4, 23.3, 24.6, 25.4, 26.1, 26.4, 27.9, 28.7, 31.0, 31.7, 32.2, 34.1, 34.9, 35.5, 36.3, 37.0, 37.7, 38.3, 40.1 and 42.1.

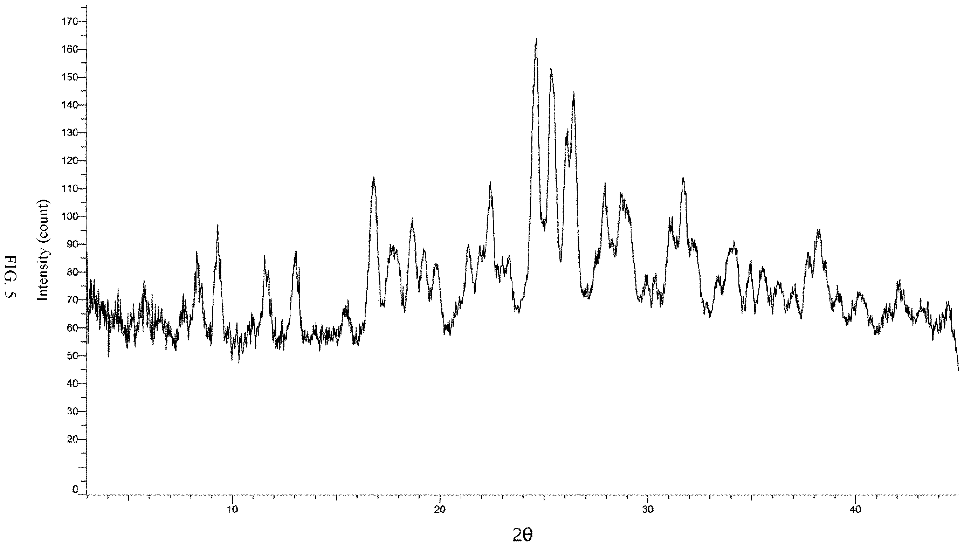

In certain embodiments, the crystalline form II of hydrobromide has an X-ray powder diffraction pattern shown in FIG. 5.

The present disclosure also provides a crystalline form of mesylate of a compound of formula (I), wherein the crystalline form is:
a crystalline form α of mesylate having an X-ray powder diffraction pattern with characteristic peaks at 2θ angles of 10.0, 16.8, 17.8, 18.4 and 20.6; or
a crystalline form β of mesylate having an X-ray powder diffraction pattern with characteristic peaks at 2θ angles of 5.9, 8.4, 14.5, 16.8, 19.8 and 26.0. In certain embodiments, the crystalline form α of mesylate has an X-ray powder diffraction pattern with characteristic peaks at 2θ angles of 7.7, 10.0, 12.9, 13.8, 14.3, 15.1, 16.8, 17.8, 18.4, 20.3, 20.6, 21.9, 23.1, 24.2, 25.3, 26.1, 26.7, 28.3, 29.0, 30.7, 35.0 and 43.1.

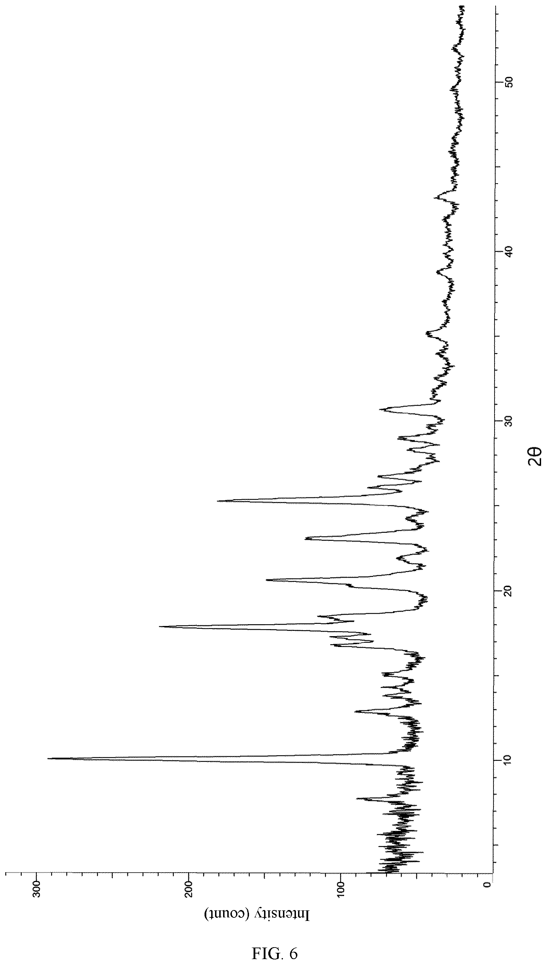

In certain embodiments, the crystalline form α of mesylate has an X-ray powder diffraction pattern shown in FIG. 6.

In certain embodiments, the crystalline form β of mesylate has an X-ray powder diffraction pattern with characteristic peaks at 2θ angles of 5.9, 8.4, 13.6, 14.5, 16.8, 18.5, 19.8, 20.9, 21.6, 23.3, 26.0, 26.7 and 27.4.

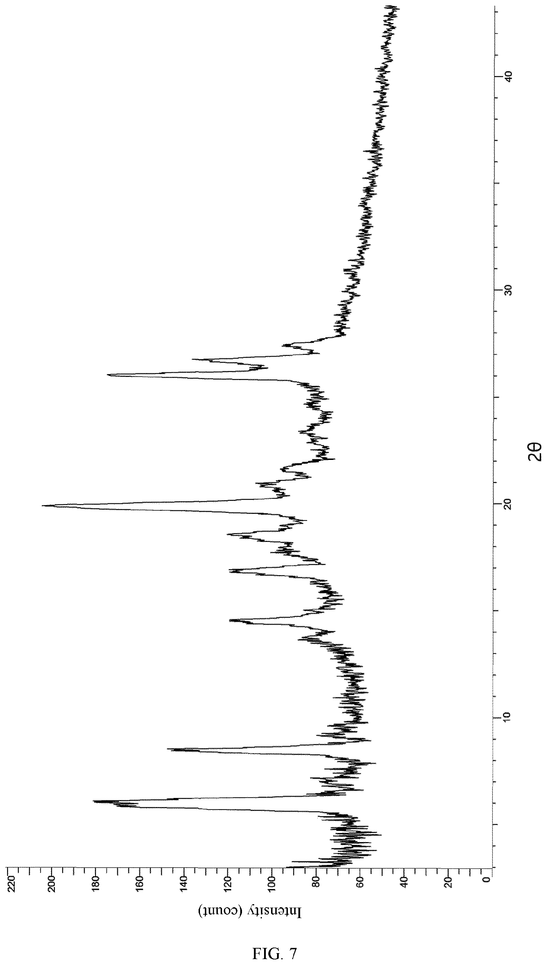

In certain embodiments, the crystalline form β of mesylate has an X-ray powder diffraction pattern shown in FIG. 7.

The present disclosure also provides a crystalline form I of maleate of a compound of formula (I), which has an X-ray powder diffraction pattern with characteristic peaks at 2θ angles of 10.1, 17.1, 18.0, 19.0 and 24.3.

In certain embodiments, the crystalline form I of maleate has an X-ray powder diffraction pattern with characteristic peaks at 2θ angles of 7.2, 9.4, 10.1, 12.8, 13.2, 14.2, 14.8, 15.7, 17.1, 18.0, 19.0, 22.0, 23.4, 24.3, 25.2, 27.5 and 29.1.

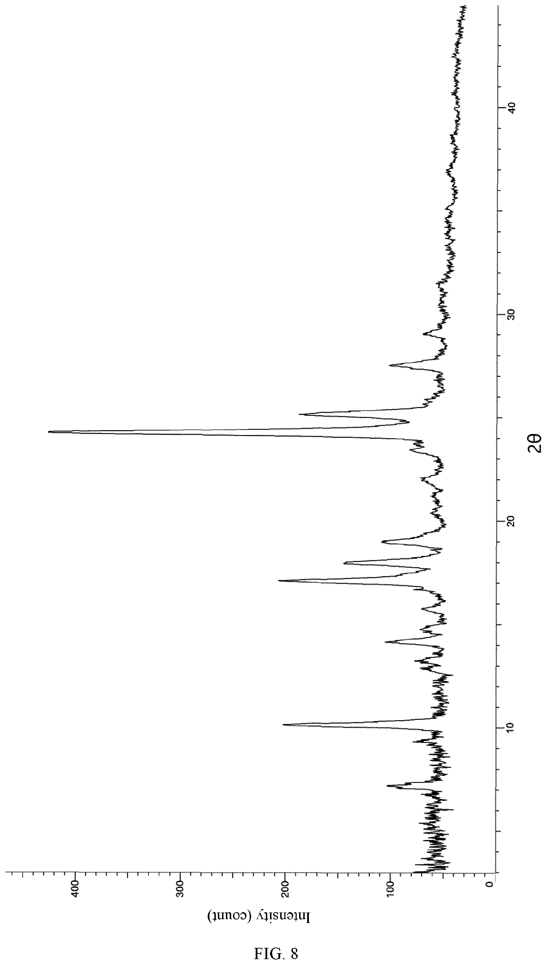

In certain embodiments, the crystalline form I of maleate has an X-ray powder diffraction pattern shown in FIG. 8.

The present disclosure also provides a crystalline form a of p-toluenesulfonate of a compound of formula (I), which has an X-ray powder diffraction pattern with characteristic peaks at 2θ angles of 6.5, 8.6, 12.0, 14.5, 21.2 and 22.2.

In certain embodiments, the crystalline form a of p-toluenesulfonate has an X-ray powder diffraction pattern with characteristic peaks at 2θ angles of 6.5, 8.6, 9.9, 12.0, 13.1, 14.5, 16.7, 18.9, 19.7, 21.2, 22.2, 24.2, 26.3 and 27.6.

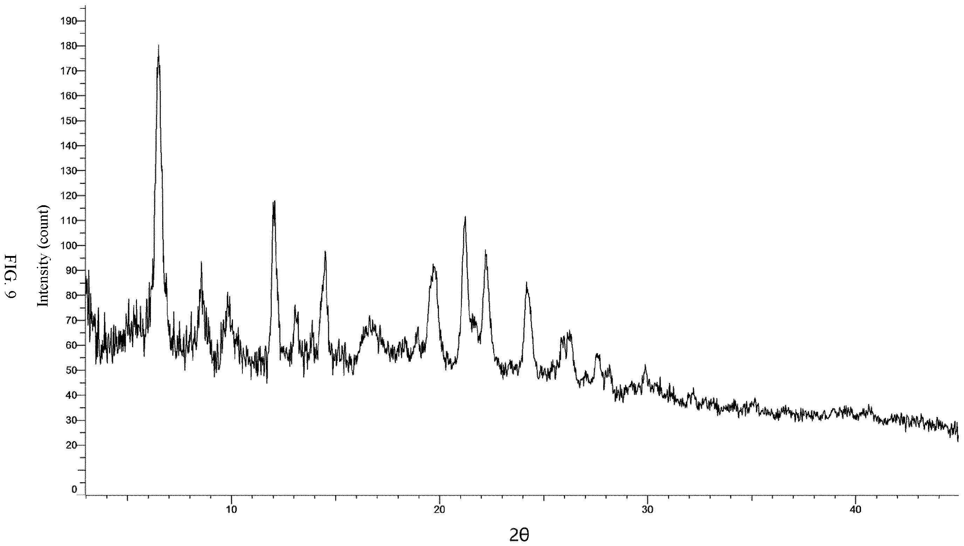

In certain embodiments, the crystalline form a of p-toluenesulfonate has an X-ray powder diffraction pattern shown in FIG. 9.

The present disclosure also provides a crystalline form a of oxalate of a compound of formula (I), which has an X-ray powder diffraction pattern with characteristic peaks at 2θ angles of 5.5, 9.1, 11.0, 13.0, 15.5, 16.5 and 20.2.

In certain embodiments, the crystalline form a of oxalate has an X-ray powder diffraction pattern with characteristic peaks at 2θ angles of 5.5, 9.1, 11.0, 13.0, 15.5, 16.5, 20.2, 22.0, 22.5, 23.1, 24.9, 26.2, 27.8 and 30.8.

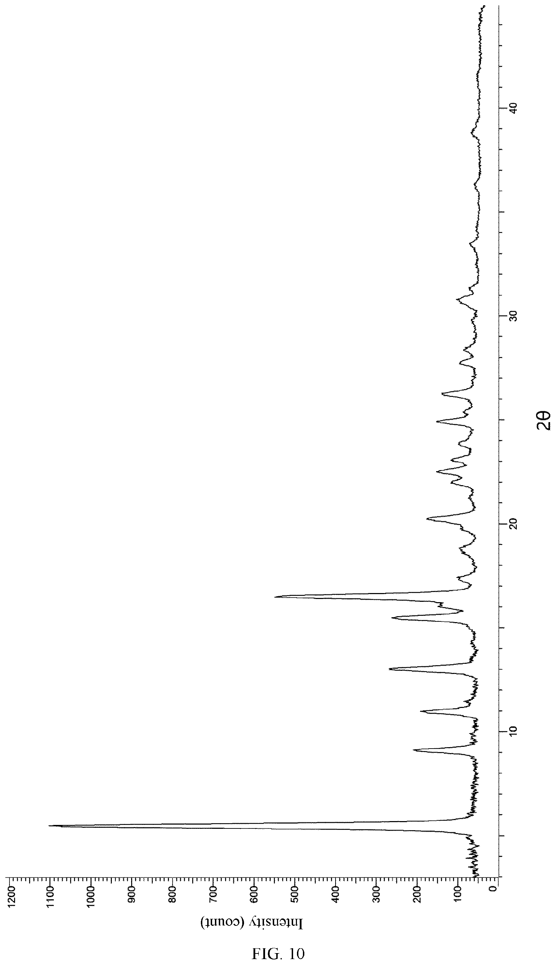

In certain embodiments, the crystalline form a of oxalate has an X-ray powder diffraction pattern shown in FIG. 10.

The present disclosure further provides a method for preparing the crystalline form a of hydrochloride of the compound of formula (I), which comprises: mixing a solution of the compound of formula (I) in methyl *tert-butyl* ether (MTBE) with hydrochloric acid, slurrying and crystallizing.

The present disclosure further provides a method for preparing the crystalline form b of hydrochloride of the compound of formula (I), which comprises: heating the hydrochloride a of the compound of formula (I) to 90 °C and collecting crystals.

The present disclosure further provides a method for preparing the crystalline form α of sulfate of the compound of formula (I), which comprises: mixing a solution comprising the compound of formula (I) and a solvent with sulfuric acid, slurrying and crystallizing, wherein the solvent is selected from the group consisting of methyl tert-butyl ether and ethyl acetate (EA)/heptane. The present disclosure further provides a method for preparing the crystalline form I of hydrobromide of the compound of formula (I), which comprises: mixing a solution comprising the compound of formula (I) and a solvent with hydrobromic acid, slurrying and crystallizing, wherein the solvent is selected from the group consisting of methyl tert-butyl ether and ethyl acetate/heptane.

The present disclosure further provides a method for preparing the crystalline form II of hydrobromide of the compound of formula (I), which comprises: mixing a solution comprising the compound of formula (I) and a solvent with hydrobromic acid, slurrying and crystallizing, wherein the solvent is selected from ethyl acetate/heptane.

The present disclosure further provides a method for preparing the crystalline form α of mesylate of the compound of formula (I), which comprises: mixing a solution comprising the compound of formula (I) and methyl tert-butyl ether with methanesulfonic acid, slurrying and crystallizing. The present disclosure further provides a method for preparing the crystalline form β of mesylate of the compound of formula (I), which comprises: mixing a solution comprising the compound of formula (I) and a solvent with methanesulfonic acid, slurrying and crystallizing, wherein the solvent is selected from the group consisting of methyl tert-butyl ether and ethyl acetate/heptane. The present disclosure further provides a method for preparing the crystalline form I of maleate of the compound of formula (I), which comprises: mixing a solution comprising the compound of formula (I) and a solvent with maleic acid, slurrying and crystallizing, wherein the solvent is selected from the group consisting of methyl *tert*-butyl ether and ethyl acetate/heptane.

The present disclosure further provides a method for preparing the crystalline form a of *p-*toluenesulfonate of the compound of formula (I), which comprises: mixing a solution comprising the compound of formula (I) and methyl *tert*-butyl ether with *p*-toluenesulfonic acid, slurrying and crystallizing.

The present disclosure further provides a method for preparing the crystalline form a of oxalate of the compound of formula (I), which comprises: mixing a solution comprising the compound of formula (I) and a solvent with oxalic acid, slurrying and crystallizing, wherein the solvent is selected from the group consisting of methyl *tert*-butyl ether and ethyl acetate/heptane.

The crystalline forms obtained by the present disclosure are subjected to structure determination and crystalline form studies through X-ray powder diffraction pattern (XRPD) and differential scanning calorimetry (DSC).

The crystallization methods for the crystalline forms in the present disclosure are conventional, such as crystallization by evaporating, crystallization by cooling or room-temperature crystallization.

The starting materials used in the method for preparing the crystalline forms disclosed herein may be any form of the compound of formula (I), including but not limited to: an amorphous form, any crystalline form, a hydrate, a solvate and the like.

The present disclosure further provides a pharmaceutical composition comprising the pharmaceutically acceptable salt of the compound of formula (I) and one or more pharmaceutically acceptable carriers or excipients.

The present disclosure further provides a pharmaceutical composition comprising the crystalline form of the pharmaceutically acceptable salt of the compound of formula (I) and one or more pharmaceutically acceptable carriers or excipients.

The present disclosure further provides a method for preparing a pharmaceutical composition, which comprises the step of: mixing the pharmaceutically acceptable salt of the compound of formula (I) with one or more pharmaceutically acceptable carriers or excipients.

The present disclosure further provides a method for preparing a pharmaceutical composition, which comprises the step of: mixing the crystalline form of the pharmaceutically acceptable salt of the compound of formula (I) with one or more pharmaceutically acceptable carriers or excipients.

The present disclosure further provides use of the pharmaceutically acceptable salt of the compound of formula (I), the crystalline form of the pharmaceutically acceptable salt or the pharmaceutical composition disclosed herein in preparing a medicament for inhibiting ATR kinase.

The present disclosure further provides use of the pharmaceutically acceptable salt of the compound of formula (I), the crystalline form of the pharmaceutically acceptable salt or the pharmaceutical composition disclosed herein in preparing a medicament for treating a hyperproliferative disease.

The present disclosure further provides use of the pharmaceutically acceptable salt of the compound of formula (I), the crystalline form of the pharmaceutically acceptable salt or the pharmaceutical composition disclosed herein in preparing a medicament for treating a tumor disease.

The tumor described herein is selected from the group consisting of melanoma, brain tumor, esophageal cancer, gastric cancer, liver cancer, pancreatic cancer, colorectal cancer, lung cancer, kidney cancer, breast cancer, cervical cancer, ovarian cancer, prostate cancer, skin cancer, neuroblastoma, neuroglioma, sarcoma, bone cancer, uterine cancer, endometrial cancer, head and neck tumor, multiple myeloma, B-cell lymphoma, polycythemia vera, leukemia, thyroid tumor, bladder cancer and gallbladder cancer.

In the specification and claims of the present application, unless otherwise specified, the scientific and technological terms used herein have meanings generally understood by those skilled in the art. However, definitions and explanations for some of the related terms are provided below for a better understanding of the present disclosure. In addition, if the definitions and explanations of the terms provided herein are not consistent with the meanings generally understood by those skilled in the art, the definitions and explanations of the terms provided herein shall prevail.

The "slurrying" described herein refers to a purification method utilizing the low solubility of the target substance and high solubility of the impurities in a solvent, which is capable of decoloring, changing the crystalline form or removing small amounts of impurities.

The "X-ray powder diffraction pattern" or "XRPD" described herein refers to a set of X-ray powder diffraction peaks obtained according to the Bragg's equation 2d sinθ = n λ (in the equation, λ is the wavelength of X-ray, the diffraction order n is any positive integer, and the first-order diffraction peak is generally taken, i.e., n = 1). When X-ray is incident on a certain atomic plane with the d-lattice plane spacing of a crystal or a partial crystal sample at a grazing angle θ (the complementary angle of incidence angle, also known as Bragg angle), the Bragg's equation can be satisfied.

The "X-ray powder diffraction pattern" or "XRPD" described herein refers to a pattern obtained by using Cu-Kα radiation in an X-ray powder diffractometer.

The "differential scanning calorimetry" or "DSC" described herein refers to measurement of the temperature difference and heat flow difference between a sample and a reference substance during a ramping or thermostatic process to characterize all the physical changes and chemical changes related to the thermal effect, and to obtain the phase change information of the sample. The "2θ" or "2θ angle" described herein refers to a diffraction angle. θ is the Bragg angle in unit ° or degree. The margin of error for 2θ may be ±0.3, ±0.2 or ±0.1.

The "interplanar spacing (d)" described herein refers to 3 selected non-parallel unit vectors (a, b and c), each of which connects two adjacent lattice points and all of which divide the lattice into juxtaposed parallelepiped units. The lattice is divided according to the determined parallelepiped unit connecting lines, giving a set of linear grids called space lattice or crystal lattice. The lattice and the crystal lattice reflect the periodicity of the crystal structure by using geometrical points and lines, respectively. The interplanar spacing (the distance between two adjacent parallel crystal planes) for different crystal planes is different; the unit is Å or Angstrom.

"Optional" or "optionally" means that the event or circumstance subsequently described may, but does not necessarily, occur, and that the description includes instances where the event or circumstance occurs or does not occur. For example, "a heterocyclyl group optionally substituted with alkyl" means that the alkyl may, but does not necessarily, exist, and that the description includes instances where the heterocyclyl group is or is not substituted with the alkyl.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, which facilitates the absorption of the active ingredient, thereby exerting biological activity.

The term "solvate" or "solvent compound" refers to a pharmaceutically acceptable solvate formed by a drug disclosed herein and one or more solvent molecules; non-limiting examples of solvent molecules include water, ethanol, methyl *tert*-butyl ether, acetone, heptane, acetonitrile, isopropanol, DMSO and ethyl acetate.

The term "carrier" for the drug disclosed herein refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

### BRIEF DESCRIPTION OF THE DRAWINGS

FIG. 1 is an XRPD pattern of the crystalline form a of hydrochloride of the compound of formula (I).
FIG. 2 is an XRPD pattern of the crystalline form b of hydrochloride of the compound of formula (I).
FIG. 3 is an XRPD pattern of the crystalline form α of sulfate of the compound of formula (I).
FIG. 4 is an XRPD pattern of the crystalline form I of hydrobromide of the compound of formula (I).
FIG. 5 is an XRPD pattern of the crystalline form II of hydrobromide of the compound of formula (I).
FIG. 6 is an XRPD pattern of the crystalline form α of mesylate of the compound of formula (I).
FIG. 7 is an XRPD pattern of the crystalline form β of mesylate of the compound of formula (I).
FIG. 8 is an XRPD pattern of the crystalline form I of maleate of the compound of formula (I).
FIG. 9 is an XRPD pattern of the crystalline form a of *p*-toluenesulfonate of the compound of formula (I).
FIG. 10 is an XRPD pattern of the crystalline form a of oxalate of the compound of formula (I).

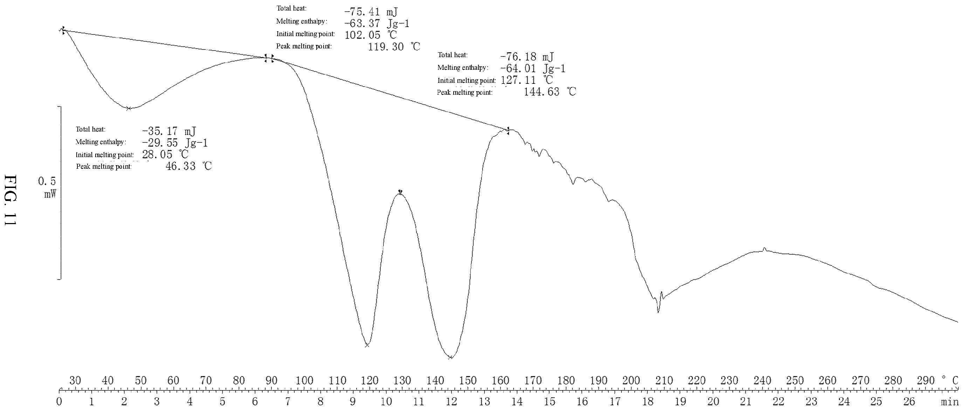

FIG. 11 is a DSC pattern of the crystalline form a of hydrochloride of the compound of formula (I).

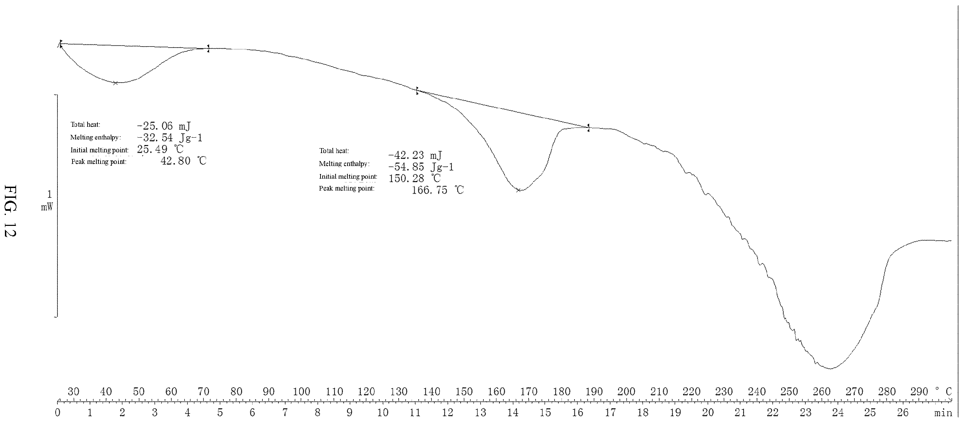

FIG. 12 is a DSC pattern of the crystalline form α of sulfate of the compound of formula (I).

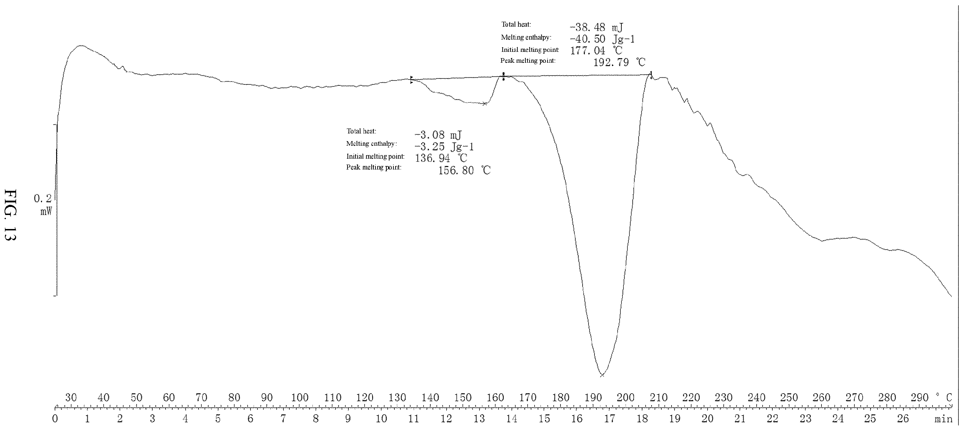

FIG. 13 is a DSC pattern of the crystalline form I of hydrobromide of the compound of formula (I).

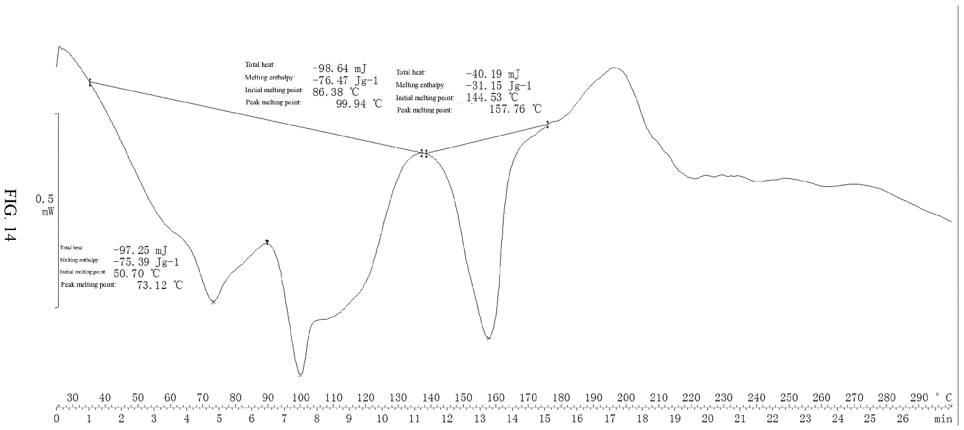

FIG. 14 is a DSC pattern of the crystalline form II of hydrobromide of the compound of formula (I).

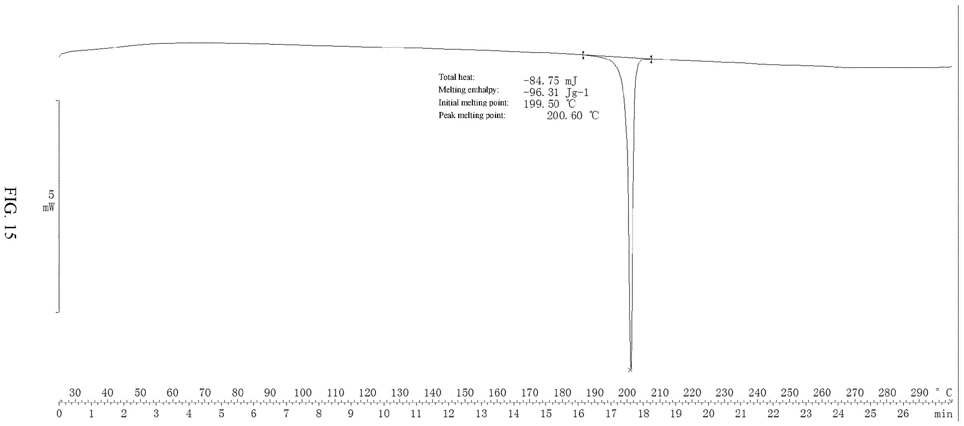

FIG. 15 is a DSC pattern of the crystalline form α of mesylate of the compound of formula (I).

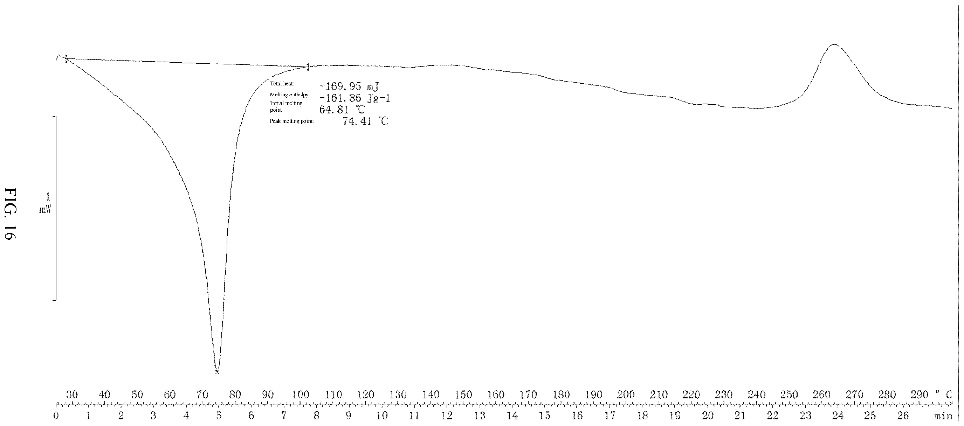

FIG. 16 is a DSC pattern of the crystalline form β of mesylate of the compound of formula (I).

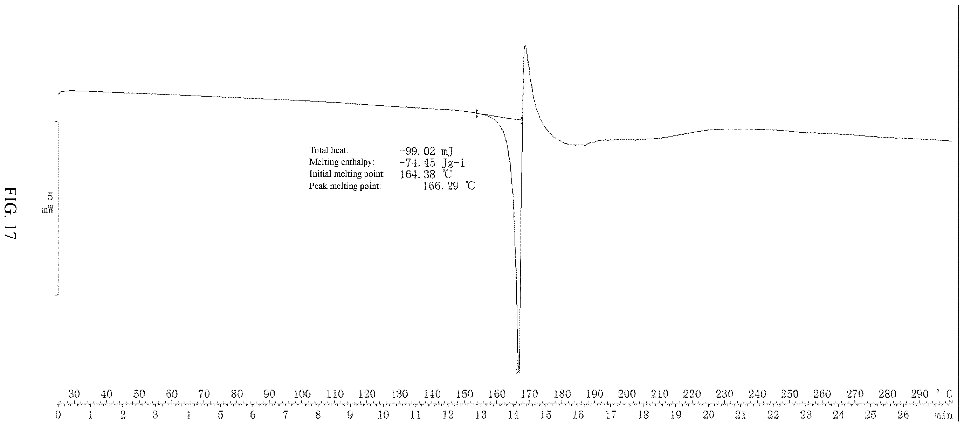

FIG. 17 is a DSC pattern of the crystalline form I of maleate of the compound of formula (I).

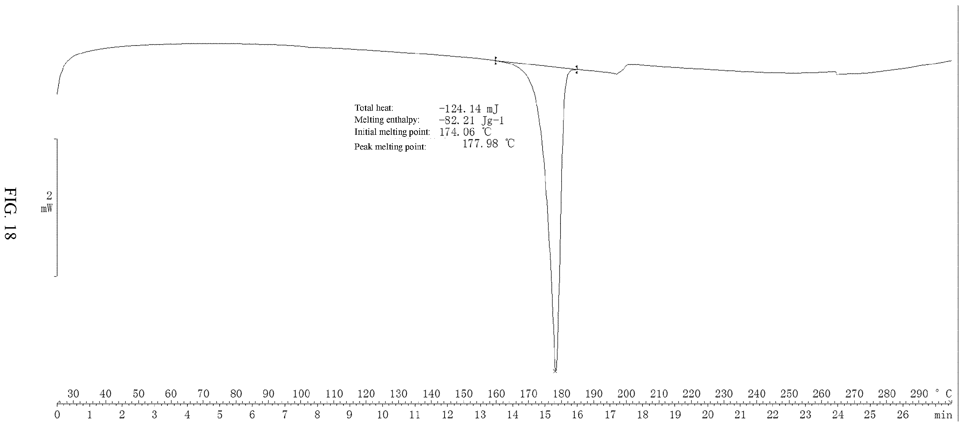

FIG. 18 is a DSC pattern of the crystalline form a of p-toluenesulfonate of the compound of formula (I).

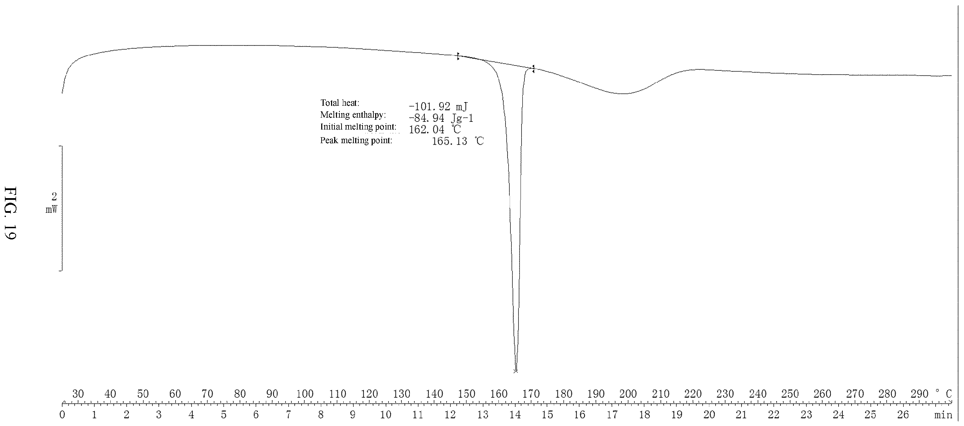

FIG. 19 is a DSC pattern of the crystalline form a of oxalate of the compound of formula (I).

### DETAILED DESCRIPTION

Hereinafter, the present disclosure will be explained in more details with reference to the examples. The examples are only used to illustrate the technical solutions of the present disclosure, rather than limit the essence and scope of the present disclosure.

The structures of compounds were determined by nuclear magnetic resonance (NMR) spectroscopy and/or mass spectrometry (MS). NMR shifts (δ) are given in 10-6 (ppm). NMR spectra were measured using a Bruker AVANCE-400 nuclear magnetic resonance instrument, with dimethyl sulfoxide-D6 (DMSO-*d*₆), chloroform-D (CDCl₃) and methanol-D4 (CD₃OD) as determination solvents, with tetramethylsilane (TMS) as internal standard.

MS analysis was performed on an Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q Exactive).

High performance liquid chromatography (HPLC) analysis was performed using the following HPLC instruments: Agilent HPLC 1260DAD, Agilent HPLC 1260VWD and Waters HPLC e2695-2489.

Chiral HPLC analyses were performed on an Agilent 1260 DAD high performance liquid chromatograph.

Preparative high performance liquid chromatography used Waters 2545-2767, Waters 2767-SQ Detecor2, Shimadzu LC-20AP and Gilson GX-281 preparative chromatographs.

Preparative chiral chromatography used a Shimadzu LC-20AP preparative chromatograph.

The CombiFlash preparative flash chromatograph used was CombiFlash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Silica gel column chromatography generally used 200- to 300-mesh silica gel (Huanghai, Yantai) as the carrier.

The mean kinase inhibition rates and IC50 values were measured using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or an HC2-SS hydrogenator.

Hydrogenation reactions generally involved 3 cycles of vacuumization and hydrogen purging. Microwave reactions were performed on a CEM Discover-S 908860 microwave reactor.

In the examples, a solution was an aqueous solution unless otherwise specified.

In the examples, reactions were conducted at room temperature, i.e., 20 °C to 30 °C, unless otherwise specified.

The monitoring of the reaction progress in the examples was conducted by thin layer chromatography (TLC). The developing solvent for reactions, the eluent system for column chromatography purification and the developing solvent system for thin layer chromatography included: A: dichloromethane/methanol system, B: n-hexane/ethyl acetate system, and C: petroleum ether/ethyl acetate system. The volume ratio of the solvents was adjusted according to the polarity of the compound, or by adding a small amount of basic or acidic reagents such as triethylamine and acetic acid.

THP refers to tetrahydropyranyl.

Test conditions of the instruments used in the test:
Differential scanning calorimeter (DSC)
Model: Mettler Toledo DSC 3+
Purging gas: nitrogen
Ramping rate: 10.0 °C/min
Temperature range: 25-300 °C

### 2. X-ray powder diffractometer (XRPD)

Model: BRUKER D8 Discover X-ray powder diffractometer
Ray: monochromatic Cu-K α ray (λ = 1.5418 Å)
Scan mode: θ/2θ, scan range (2θ range): 3-50°
Voltage: 40 kV, current: 40 mA

### 3. Ion chromatography

Model: DIONEX INTEGRION HPIC ion chromatograph, USA
Detection mode: conductance; separation column: DionexIonPacTM-AS11-HC
Rinsing solution: EGC-500-KOH
Flow rate: 1.4 mL/min

### Example 1

### (R)-2-methyl-2-(1-methyl-5-(3-methylmorpholino)-3-(1H-pyrazol-3-yl)-1H-pyrazolo[4,3-b]pyridin-7-yl)propanenitrile I

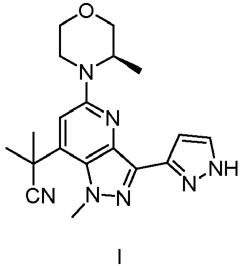

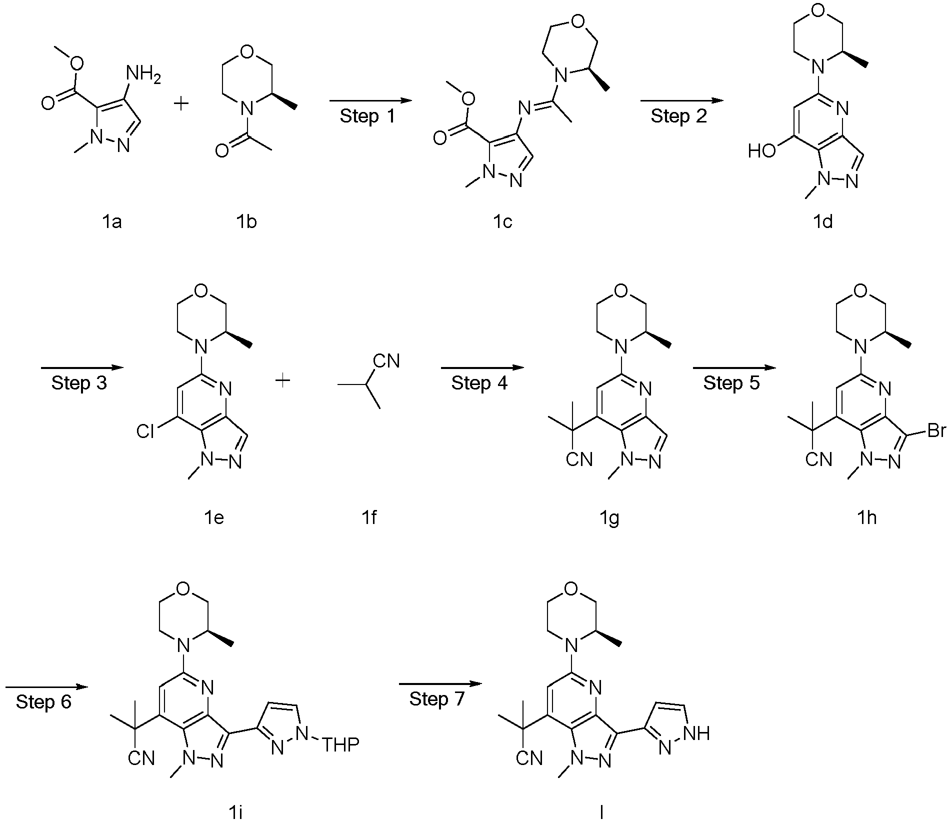

### Step 1

### Methyl (R,E)-1-methyl-4-((1-(3-methylmorpholino)ethylidene)amino)-1H-pyrazole-5-carboxylate 1c

Compound (R)-1-(3-methylmorpholinyl)ethan-1-one 1b (2.5 g, 17.7 mmol, prepared by the method disclosed for intermediate-1 in the Example on page 86 of the Patent Application No. WO2016020320A1) was dissolved in 1,2-dichloroethane, and the mixture was cooled in an ice/water bath in an argon atmosphere. Phosphorus oxychloride (7.4 g, 48.3 mmol) was added dropwise and slowly, and then the resulting mixture was stirred at room temperature for 30 min. Compound methyl 4-amino-1-methyl-1*H*-pyrazole-5-formate 1a (2.5 g, 16.1 mmol, Jiangsu Aikon) was added. The reaction mixture was heated to 80 °C and stirred for 2 h. The reaction mixture was then cooled to room temperature and concentrated at reduced pressure. The residue was diluted with dichloromethane (200 mL), and the dilution was cooled in an ice/water bath. Saturated sodium bicarbonate solution was added dropwise to neutralize the dilution to pH 8 to 9. The organic phase was washed with saturated brine (50 mL), dried over anhydrous sodium sulfate and filtered. The filtrate was mixed with silica gel and then purified by silica gel column chromatography with eluent system C to obtain the target compound 1c (4.8 g, 94% yield).
MS m/z (ESI): 281.2 [M+1]

### Step 2

### (R)-1-methyl-5-(3-methylmorpholino)-1H-pyrazolo[4,3-b]pyridin-7-ol 1d

Compound 1c (2.6 g, 9.3 mmol) was dissolved in tetrahydrofuran (20 mL), and the mixture was cooled in an ice/water bath. Lithium bis(trimethylsilyl)amide (27.8 mL, a 1 M solution in tetrahydrofuran, 27.8 mmol) was added slowly and the reaction mixture was reacted at 0 °C for 1 h. The reaction was quenched with methanol (10 mL). The residue was mixed with silica gel and then purified by silica gel column chromatography with eluent system A to obtain the target compound 1d (400 mg, 55.8% yield).
MS m/z (ESI): 249.0 [M+1]

### Step 3

### (R)-4-(7-chloro-1-methyl-1H-pyrazolo[4,3-b]pyridin-5-yl)-3-methylmorpholine 1e

Compound 1d (400 mg, 1.6 mmol) was dissolved in phosphorus oxychloride (3.0 mL). The mixture was heated to 90 °C and stirred for 2.0 h. The reaction mixture was cooled to room temperature and concentrated at reduced pressure. The residue was diluted with dichloromethane (50 mL), and the dilution was cooled in an ice/water bath. Saturated sodium bicarbonate solution was added to neutralize the dilution to pH 8 to 9. The mixture was stirred for 0.5 h for reaction and left to stand for separation. The organic phase was collected, washed with saturated brine (50 mL), dried over anhydrous sodium sulfate and filtered. The filtrate was mixed with silica gel and then purified by silica gel column chromatography with eluent system C to obtain the target compound 1e (240 mg, 56% yield).
MS m/z (ESI): 267.0 [M+1]

### Step 4

### (R)-2-methyl-2-(1-methyl-5-(3-methylmorpholino)-1H-pyrazolo[4,3-b]pyridin-7-yl)propanenitrile 1g

Compound 1e (240 mg, 0.91 mmol) and compound isobutyronitrile 1f (620 mg, 8.9 mmol, Shanghai Bide) were dissolved in tetrahydrofuran (30 mL), and the mixture was cooled in a dry ice/acetone bath in an argon atmosphere. Lithium bis(trimethylsilyl)amide (8.9 mL, a 1 M solution in tetrahydrofuran, 8.9 mmol) was added dropwise. The mixture was stirred at a low temperature for 0.5 h, naturally warmed to room temperature and stirred for 1 h. The reaction was quenched with water. The organic phase was washed with saturated brine (50 mL), dried over anhydrous sodium sulfate and filtered. The filtrate was concentrated at reduced pressure, and the residue was purified by silica gel column chromatography with eluent system C to obtain the target compound 1g (200 mg, 74% yield).
MS m/z (ESI): 300.1 [M+1]

### Step 5

### (R)-2-(3-bromo-1-methyl-5-(3-methylmorpholino)-1H-pyrazolo[4,3-b]pyridin-7-yl)-2-methylpropanenitrile 1h

Compound 1g (200 mg, 0.67 mmol) was dissolved in 1,4-dioxane (5 mL), and a solution of sodium hydroxide (0.66 mL, 2 M, 1.32 mmol) was added. The mixture was cooled in an ice/water bath, and then bromine (427 mg, 2.67 mmol) was added. The reaction mixture was stirred at a low temperature for 10 min, naturally warmed to room temperature and stirred for 1 h for reaction. Ethyl acetate was added for dilution. The organic phase was washed with saturated sodium thiosulfate solution and saturated sodium chloride solution, dried over anhydrous sodium sulfate and filtered. The filtrate was concentrated at reduced pressure, and the residue was purified by silica gel column chromatography with eluent system C to obtain the target compound 1h (140 mg, 55% yield).
MS m/z (ESI): 377.9 [M+1]

### Step 6

### 2-methyl-2-(1-methyl-5-((R)-3-methylmorpholino)-3-(1-(tetrahydro-2H-pyran-2-yl)-1H-pyrazol-3-yl)-1H-pyrazolo[4,3-b]pyridin-7-yl)propanenitrile 1i

Compound 1h (20 mg, 0.05 mmol), tetrakis(triphenylphosphine)palladium(0) (18 mg, 0.015 mmol), sodium carbonate (11 mg, 0.10 mmol) and 1-(tetrahydro-2*H*-pyran-2-yl)-3-(4,4,5,5-tetramethyl-1,3,2-dioxaborolan-2-yl)-1*H*-pyrazole (29 mg, 0.10 mmol, Bide, Shanghai) were dissolved in ethylene glycol dimethyl ether (4 mL). Water (1 mL) was added. In an argon atmosphere, the reaction mixture was heated by microwave to 120 °C and reacted for 1 h. The reaction mixture was cooled to room temperature, and then water (20 mL) was added. Ethyl acetate (20 mL × 3) was added for extraction. The organic phases were combined, concentrated at reduced pressure, washed with saturated sodium chloride solution, dried over anhydrous sodium sulfate and filtered. The filtrate was concentrated at reduced pressure, and the residue was purified by silica gel column chromatography with eluent system C to obtain the target compound 1i (20 mg, 84% yield).
MS m/z (ESI): 450.1 [M+1]

### Step 7

### (R)-2-methyl-2-(1-methyl-5-(3-methylmorpholino)-3-(1H-pyrazol-3-yl)-1H-pyrazolo[4,3-b]pyridin-7-yl)propanenitrile I

Compound 1i (20 mg, 0.04 mmol) was dissolved in dichloromethane (5 mL). Trifluoroacetic acid (5 mL) was added dropwise, and the reaction mixture was stirred for 4 h for reaction. The reaction mixture was concentrated at reduced pressure, and a 7 M solution of ammonia in methanol was added dropwise to adjust the pH to 8 to 9. The resulting mixture was concentrated at reduced pressure, and the residue was purified by silica gel column chromatography with eluent system A to obtain the target compound I (7.0 mg, 43% yield).
MS m/z (ESI): 366.0 [M+1]
1H NMR (400 MHz, CD3OD):δ 7.58 (s, 1H), 7.03 (s, 1H), 6.86 (s, 1H), 4.39 (s, 4H), 4.04 - 3.82 (m, 2H), 3.74 (s, 2H), 3.58 (td, 1H), 3.26 (dd, 1H), 1.88 (d, 6H), 1.19 (d, 3H).

### Example 2

An MTBE solution (0.25 mL) containing the compound of formula (I) (about 10 mg) obtained in Example 1 was mixed with a solution of hydrochloric acid in ethanol (22.5 µL, 1.2 mol/L), and the mixture was subjected to slurrying. The solid was separated out by centrifugation and dried *in vacuo* to obtain the product. The product was identified by X-ray powder diffraction as the crystalline form a of hydrochloride. The XRPD pattern is shown in FIG. 1, and the positions of characteristic peaks are shown in Table 1.

**Table 1**

| Peak No. | 2θ[°] | d[Å] | Relative intensity |
|---|---|---|---|
| 1 | 6.046 | 14.60688 | 100.0% |
| 2 | 8.275 | 10.67578 | 70.3% |
| 3 | 9.061 | 9.75196 | 11.5% |
| 4 | 12.086 | 7.31702 | 24.4% |
| 5 | 14.302 | 6.18784 | 16.6% |
| 6 | 14.896 | 5.94233 | 12.7% |
| 7 | 16.727 | 5.29583 | 18.0% |
| 8 | 18.327 | 4.83701 | 25.1% |
| 9 | 19.449 | 4.56045 | 7.2% |
| 10 | 23.476 | 3.7864 | 26.6% |
| 11 | 24.291 | 3.66122 | 6.5% |
| 12 | 26.291 | 3.38703 | 23.0% |
| 13 | 26.704 | 3.33559 | 46.3% |

### Example 3

After the crystalline form a of hydrochloride of the compound of formula (I) was heated to 90 °C, the transformation of the crystalline form was detected. The resulting product was defined as the crystalline form b of hydrochloride. The XRPD pattern is shown in FIG. 2, and the positions of characteristic peaks are shown in Table 2.

**Table 2**

| Peak No. | 2θ[°] | d[Å] | Relative intensity |
|---|---|---|---|
| 1 | 6.035 | 14.63376 | 100.0 % |
| 2 | 8.440 | 10.46807 | 6.2 % |
| 3 | 8.954 | 9.86812 | 6.8 % |
| 4 | 12.082 | 7.31939 | 33.9 % |
| 5 | 16.467 | 5.37902 | 3.7 % |
| 6 | 18.210 | 4.86789 | 12.7 % |
| 7 | 23.553 | 3.77431 | 7.9 % |
| 8 | 24.354 | 3.65182 | 7.4 % |
| 9 | 26.234 | 3.39423 | 6.4 % |
| 10 | 29.466 | 3.02888 | 3.2 % |
| 11 | 33.948 | 2.63856 | 3.6 % |
| 12 | 35.535 | 2.52426 | 2.4 % |

### Example 4

An MTBE solution (0.25 mL) containing the compound of formula (I) (about 10 mg) was mixed with a solution of sulfuric acid in ethanol (14.7 µL, 1.8 mol/L), and the mixture was subjected to slurrying. The solid was separated out by centrifugation and dried *in vacuo* to obtain the product. The product was identified by X-ray powder diffraction as the crystalline form α of sulfate, and the sulfate ion content of the product was 17.9% as detected by ion chromatography. The XRPD pattern is shown in FIG. 3, and the positions of characteristic peaks are shown in Table 3.

**Table 3**

| Peak No. | 2θ[°] | d[Å] | Relative intensity |
|---|---|---|---|
| 1 | 5.817 | 15.18018 | 100.0% |
| 2 | 7.586 | 11.64367 | 67.3% |
| 3 | 13.667 | 6.47401 | 11.7% |
| 4 | 15.378 | 5.75717 | 17.3% |
| 5 | 16.440 | 5.38755 | 7.0% |
| 6 | 16.920 | 5.23576 | 5.7% |
| 7 | 18.011 | 4.92121 | 4.9% |
| 8 | 18.534 | 4.78342 | 8.1% |
| 9 | 19.168 | 4.62657 | 8.3% |
| 10 | 20.393 | 4.3514 | 25.2% |
| 11 | 22.982 | 3.86666 | 5.9% |
| 12 | 23.942 | 3.71384 | 2.5% |
| 13 | 25.876 | 3.44038 | 11.2% |

### Example 5

An MTBE solution (0.25 mL) containing the compound of formula (I) (about 10 mg) was mixed with a solution of hydrobromic acid in ethanol (36 µL, 0.75 mol/L), and the mixture was subjected to slurrying. The solid was separated out by centrifugation and dried *in vacuo* to obtain the product. The product was identified by X-ray powder diffraction as the crystalline form I of hydrobromide. The XRPD pattern is shown in FIG. 4, and the positions of characteristic peaks are shown in Table 4.

**Table 4**

| Peak No. | 2θ[°] | d[Å] | Relative intensity |
|---|---|---|---|
| 1 | 5.958 | 14.82113 | 24.0% |
| 2 | 8.141 | 10.85125 | 100.0% |
| 3 | 14.700 | 6.02116 | 31.5% |
| 4 | 17.273 | 5.12962 | 12.4% |
| 5 | 18.843 | 4.70562 | 7.1% |
| 6 | 21.983 | 4.04009 | 18.5% |
| 7 | 25.864 | 3.44206 | 49.9% |
| 8 | 26.955 | 3.30515 | 24.5% |
| 9 | 27.783 | 3.20844 | 6.5% |

### Example 6

An EA/heptane solution (1:1, 0.25 mL) containing the compound of formula (I) (about 10 mg) was mixed with a solution of hydrobromic acid in ethanol (72 µL, 0.75 mol/L), and the mixture was subjected to slurrying. The solid was separated out by centrifugation and dried *in vacuo* to obtain the product. The product was identified by X-ray powder diffraction as the crystalline form II of hydrobromide. The XRPD pattern is shown in FIG. 5, and the positions of characteristic peaks are shown in Table 5.

**Table 5**

| Peak No. | 2θ[°] | d[Å] | Relative intensity |
|---|---|---|---|
| 1 | 8.243 | 10.71769 | 15.5% |
| 2 | 9.287 | 9.5153 | 43.0% |
| 3 | 11.627 | 7.60497 | 24.8% |
| 4 | 13.016 | 6.79628 | 27.7% |
| 5 | 15.497 | 5.71332 | 4.1% |
| 6 | 16.803 | 5.27195 | 58.0% |
| 7 | 17.580 | 5.04082 | 22.4% |
| 8 | 18.664 | 4.7504 | 34.5% |
| 9 | 19.268 | 4.60289 | 23.2% |
| 10 | 19.770 | 4.48696 | 18.9% |
| 11 | 21.315 | 4.16526 | 20.9% |
| 12 | 22.429 | 3.96077 | 44.8% |
| 13 | 23.290 | 3.8163 | 11.1% |
| 14 | 24.607 | 3.61491 | 100.0% |
| 15 | 25.391 | 3.50503 | 83.3% |
| 16 | 26.127 | 3.40789 | 59.3% |
| 17 | 26.419 | 3.37088 | 70.5% |
| 18 | 27.922 | 3.19277 | 34.6% |
| 19 | 28.748 | 3.10289 | 33.1% |
| 20 | 30.975 | 2.88474 | 15.8% |
| 21 | 31.722 | 2.8185 | 44.1% |
| 22 | 32.232 | 2.77506 | 16.4% |
| 23 | 34.063 | 2.62993 | 15.9% |
| 24 | 34.889 | 2.56954 | 10.2% |
| 25 | 35.535 | 2.52426 | 8.6% |
| 26 | 36.325 | 2.47115 | 1.8% |
| 27 | 37.044 | 2.42487 | 1.5% |
| 28 | 37.726 | 2.38257 | 18.0% |
| 29 | 38.336 | 2.34602 | 23.4% |
| 30 | 40.132 | 2.2451 | 4.7% |
| 31 | 42.107 | 2.14425 | 12.5% |

### Example 7

An MTBE solution (0.25 mL) containing the compound of formula (I) (about 10 mg) was mixed with a solution of methanesulfonic acid in ethanol (17.7 µL, 1.5 mol/L), and the mixture was subjected to slurrying. The solid was separated out by centrifugation and dried *in vacuo* to obtain the product. The product was identified by X-ray powder diffraction as the crystalline form α of mesylate, and the mesylate ion content of the product was 20.0% as detected by ion chromatography. The XRPD pattern is shown in FIG. 6, and the positions of characteristic peaks are shown in Table 6.

**Table 6**

| Peak No. | 2θ[°] | d[Å] | Relative intensity |
|---|---|---|---|
| 1 | 7.688 | 11.49034 | 12.1% |
| 2 | 10.04 | 8.80284 | 100.0% |
| 3 | 12.864 | 6.87629 | 13.8% |
| 4 | 13.76 | 6.43046 | 6.8% |
| 5 | 14.285 | 6.19512 | 4.3% |
| 6 | 15.073 | 5.87296 | 5.9% |
| 7 | 16.765 | 5.28391 | 21.0% |
| 8 | 17.241 | 5.13914 | 20.5% |
| 9 | 17.827 | 4.97153 | 68.4% |
| 10 | 18.443 | 4.80693 | 25.3% |
| 11 | 20.303 | 4.37052 | 18.0% |
| 12 | 20.586 | 4.311 | 41.1% |
| 13 | 21.859 | 4.06267 | 5.7% |
| 14 | 23.067 | 3.85259 | 30.2% |
| 15 | 24.224 | 3.67125 | 3.0% |
| 16 | 25.294 | 3.51829 | 52.9% |
| 17 | 26.084 | 3.41347 | 13.6% |
| 18 | 26.706 | 3.3353 | 12.0% |
| 19 | 28.251 | 3.15632 | 4.8% |
| 20 | 28.977 | 3.07891 | 7.1% |
| 21 | 30.669 | 2.91282 | 14.2% |
| 22 | 35.037 | 2.55899 | 2.7% |
| 23 | 43.137 | 2.09541 | 4.4% |

### Example 8

An MTBE solution (0.25 mL) containing the compound of formula (I) (about 10 mg) was mixed with a solution of methanesulfonic acid in ethanol (35.4 µL, 1.5 mol/L), and the mixture was subjected to slurrying. The solid was separated out by centrifugation and dried *in vacuo* to obtain the product. The product was identified by X-ray powder diffraction as the crystalline form β of mesylate. The XRPD pattern is shown in FIG. 7, and the positions of characteristic peaks are shown in Table 7.

**Table 7**

| Peak No. | 2θ[°] | d[Å] | Relative intensity |
|---|---|---|---|
| 1 | 5.879 | 15.02053 | 97.2% |
| 2 | 8.444 | 10.46268 | 66.1% |
| 3 | 13.628 | 6.49225 | 15.4% |
| 4 | 14.49 | 6.10822 | 36.8% |
| 5 | 16.838 | 5.26112 | 30.1% |
| 6 | 18.531 | 4.78422 | 26.7% |
| 7 | 19.844 | 4.4706 | 100.0% |
| 8 | 20.879 | 4.25125 | 18.0% |
| 9 | 21.569 | 4.11669 | 10.4% |
| 10 | 23.33 | 3.80982 | 3.2% |
| 11 | 25.982 | 3.42664 | 83.6% |
| 12 | 26.681 | 3.33848 | 42.3% |
| 13 | 27.404 | 3.25198 | 16.2% |

### Example 9

An MTBE solution (0.4 mL) containing the compound of formula (I) (about 10 mg) was mixed with a solution of maleic acid in ethanol (30 µL, 1 mol/L), and the mixture was subjected to slurrying. The solid was separated out by centrifugation and dried *in vacuo* to obtain the product. The product was identified by X-ray powder diffraction as the crystalline form I of maleate, and the maleate ion content of the product was 23.7% as detected by ion chromatography. The XRPD pattern is shown in FIG. 8, and the positions of characteristic peaks are shown in Table 8.

**Table 8**

| Peak No. | 2θ[°] | d[Å] | Relative intensity |
|---|---|---|---|
| 1 | 7.177 | 12.30766 | 13.5% |
| 2 | 9.353 | 9.44786 | 5.0% |
| 3 | 10.136 | 8.71971 | 40.7% |
| 4 | 12.835 | 6.89171 | 4.6% |
| 5 | 13.23 | 6.68691 | 7.1% |
| 6 | 14.162 | 6.24878 | 13.2% |
| 7 | 14.773 | 5.99158 | 4.4% |
| 8 | 15.706 | 5.63761 | 3.5% |
| 9 | 17.096 | 5.18242 | 40.6% |
| 10 | 17.976 | 4.93059 | 22.9% |
| 11 | 18.987 | 4.67032 | 14.0% |
| 12 | 21.988 | 4.03922 | 3.3% |
| 13 | 23.388 | 3.80052 | 5.0% |
| 14 | 24.262 | 3.66546 | 100.0% |
| 15 | 25.159 | 3.53689 | 32.6% |
| 16 | 27.526 | 3.23778 | 12.1% |
| 17 | 29.059 | 3.07041 | 3.5% |

### Example 10

An MTBE solution (0.4 mL) containing the compound of formula (I) (about 10 mg) was mixed with a solution of *p*-toluenesulfonic acid in ethanol (30 µL, 1 mol/L), and the mixture was subjected to slurrying. The solid was separated out by centrifugation and dried *in vacuo* to obtain the product. The product was identified by X-ray powder diffraction as the crystalline form a of *p*-toluenesulfonate, and the *p*-toluenesulfonate ion content of the product was 34.6% as detected by ion chromatography. The XRPD pattern is shown in FIG. 9, and the positions of characteristic peaks are shown in Table 9.

**Table 9**

| Peak No. | 2θ[°] | d[Å] | Relative intensity |
|---|---|---|---|
| 1 | 6.493 | 13.60161 | 100.0% |
| 2 | 8.561 | 10.32026 | 31.4% |
| 3 | 9.856 | 8.96724 | 18.0% |
| 4 | 12.047 | 7.34038 | 50.7% |
| 5 | 13.050 | 6.77846 | 17.8% |
| 6 | 14.466 | 6.11801 | 29.9% |
| 7 | 16.711 | 5.30076 | 5.7% |
| 8 | 18.901 | 4.69136 | 0.3% |
| 9 | 19.729 | 4.49621 | 27.8% |
| 10 | 21.214 | 4.18488 | 44.4% |
| 11 | 22.234 | 3.99500 | 33.9% |
| 12 | 24.224 | 3.67124 | 24.4% |
| 13 | 26.259 | 3.39108 | 12.3% |
| 14 | 27.623 | 3.22665 | 6.5% |

### Example 11

An MTBE solution (0.4 mL) containing the compound of formula (I) (about 10 mg) was mixed with a solution of oxalic acid in ethanol (30 µL, 1 mol/L), and the mixture was subjected to slurrying. The solid was separated out by centrifugation and dried *in vacuo* to obtain the product. The product was identified by X-ray powder diffraction as the crystalline form a of oxalate, and the oxalate ion content of the product was 10.9% as detected by ion chromatography. The XRPD pattern is shown in FIG. 10, and the positions of characteristic peaks are shown in Table 10.

**Table 10**

| Peak No. | 2θ[°] | d[Å] | Relative intensity |
|---|---|---|---|
| 1 | 5.466 | 16.15426 | 100.0% |
| 2 | 9.103 | 9.70699 | 13.9% |
| 3 | 10.963 | 8.06377 | 11.9% |
| 4 | 13.015 | 6.79671 | 20.2% |
| 5 | 15.477 | 5.72065 | 18.8% |
| 6 | 16.091 | 5.50378 | 6.7% |
| 7 | 16.504 | 5.36689 | 46.1% |
| 8 | 17.357 | 5.10511 | 3.1% |
| 9 | 18.722 | 4.73592 | 2.5% |
| 10 | 20.239 | 4.38422 | 10.5% |
| 11 | 21.999 | 4.03715 | 3.9% |
| 12 | 22.516 | 3.94572 | 7.1% |
| 13 | 23.077 | 3.85101 | 3.8% |
| 14 | 23.851 | 3.72768 | 2.2% |
| 15 | 24.916 | 3.57072 | 8.0% |
| 16 | 26.24 | 3.39354 | 6.7% |
| 17 | 27.751 | 3.21207 | 3.0% |
| 18 | 30.756 | 2.90474 | 3.4% |

### Example 12

The crystalline form α of mesylate, the crystalline form α of sulfate, the crystalline form I of maleate, the crystalline form a of *p*-toluenesulfonate and the crystalline form a of oxalate were stored in sealed aluminum foil bags and subjected to stability test under the conditions of -20 °C, 4 °C, 25 °C/60% RH and 40 °C/75% RH. The results are shown below.

**Table 11**

| | | | | | | |
|---|---|---|---|---|---|---|
| Crystalline form α of mesylate | Storage condition | Purity (%) | | | Crystalline form | |
| | | Initial | 1 month | 2 months | 3 months | |
| | -20°C | 98.72 | 98.73 | 98.69 | 98.61 | Unchanged |
| | 4°C | | 98.66 | 98.65 | 98.64 | Unchanged |
| | 25°C/60%RH | 98.49 | 98.53 | 98.54 | 98.62 | Unchanged |
| | 40°C/75%RH | | 97.00 | 97.56 | 95.66 | Unchanged |
| Crystalline form α of sulfate | Storage condition | Purity (%) | | | | Crystalline form |
| | | Initial | 1 month | 2 months | | |
| | -20°C | 97.55 | 97.62 | 97.56 | | Unchanged |
| | 4°C | | 97.07 | 97.29 | | Unchanged |
| | 25°C/60%RH | 97.70 | 93.00 | 92.90 | | Unchanged |
| | 40°C/75%RH | | 89.37 | 83.97 | | Unchanged |
| Crystalline form I of maleate | Storage condition | Purity (%) | | | | Crystalline form |
| | | Initial | 1 month | 2 months | 3 months | |
| | -20°C | 98.97 | 99.04 | 99.00 | 99.00 | Unchanged |
| | 4°C | | 98.99 | 98.99 | 99.00 | Unchanged |
| | 25°C/60%RH | | 98.84 | 98.82 | 98.77 | Unchanged |
| | 40°C/75%RH | | 98.59 | 98.26 | 98.30 | Unchanged |
| Crystalline form a of *p-*toluenesulfonate | Storage condition | Purity (%) | | | | Crystalline form |
| | | Initial | 1 month | 2 months | 3 months | |
| | -20°C | 98.69 | 98.46 | 98.46 | 98.66 | Unchanged |
| | 4°C | | 98.13 | 98.19 | 98.17 | Unchanged |
| | 25°C/60%RH | | 98.41 | 98.07 | 98.18 | Unchanged |
| | 40°C/75%RH | | 97.31 | 96.90 | 95.57 | Unchanged |
| Crystalline form a of oxalate | Storage condition | Purity (%) | | | | Crystalline form |
| | | Initial | 1 month | 2 months | 3 months | |
| | -20°C | 99.57 | 99.57 | 99.51 | 99.46 | Unchanged |
| | 4°C | | 99.54 | 99.60 | 99.55 | Unchanged |
| | 25°C/60%RH | | 99.53 | 99.58 | 99.52 | Unchanged |
| | 40°C/75%RH | | 99.40 | 99.39 | 99.40 | Unchanged |

The long-term/accelerated stability test showed that: all salt forms exhibited good physical stability; for chemical stability, the crystalline form a of oxalate was relatively stable, the crystalline form α of mesylate and the crystalline form I of maleate were slightly degraded under the conditions of 40 °C and 75% RH, and the crystalline form α of sulfate and the crystalline form a of *p*-toluenesulfonate showed slightly poor stability.

### Example 13

An ethanol/water solution (V/V, 9:1, 0.2 mL) containing the compound of formula (I) (about 10 mg) was mixed with a solution of phosphoric acid in ethanol (18.5 µL, 1.5 mol/L), and the mixture was subjected to slurrying. The solid was separated out by centrifugation and dried *in vacuo* to obtain the product. The product was identified by X-ray powder diffraction as phosphate in amorphous form.

### Example 14

An MTBE solution (0.25 mL) containing the compound of formula (I) (about 10 mg) was mixed with a solution of formic acid in ethanol (10.2 µL, 2.7 mol/L), and the mixture was subjected to slurrying. The solid was separated out by centrifugation and dried *in vacuo* to obtain the product. The product was identified by X-ray powder diffraction as formate in amorphous form.

### Example 15

An ethyl acetate/heptane solution (V/V, 9:1, 0.25 mL) containing the compound of formula (I) (about 10 mg) was mixed with a solution of acetic acid in ethanol (15.4 µL, 1.8 mol/L), and the mixture was subjected to slurrying. The solid was separated out by centrifugation and dried *in vacuo* to obtain the product. The product was identified by X-ray powder diffraction as acetate in amorphous form.

### Example 16

An ethyl acetate/heptane solution (V/V, 9:1, 0.25 mL) containing the compound of formula (I) (about 10 mg) was mixed with a solution of succinic acid in ethanol (33 µL, 0.9 mol/L), and the mixture was subjected to slurrying. The solid was separated out by centrifugation and dried *in vacuo* to obtain the product. The product was identified by X-ray powder diffraction as succinate in amorphous form.

### Example 17

An ethanol/water solution (V/V, 9:1, 0.4 mL) containing the compound of formula (I) (about 10 mg) was mixed with a solution of fumaric acid in ethanol (60 µL, 0.5 mol/L), and the mixture was subjected to slurrying. The solid was separated out by centrifugation and dried *in vacuo* to obtain the product. The product was identified by X-ray powder diffraction as fumarate in amorphous form.

### Example 18

An MTBE solution (0.4 mL) containing the compound of formula (I) (about 10 mg) was mixed with a solution of citric acid in ethanol (30 µL, 1 mol/L), and the mixture was subjected to slurrying. The solid was separated out by centrifugation and dried *in vacuo* to obtain the product. The product was identified by X-ray powder diffraction as citrate in amorphous form.

### Example 19

An ethyl acetate/heptane solution (V/V, 9:1, 0.25 mL) containing the compound of formula (I) (about 10 mg) was mixed with a solution of malic acid in ethanol (30 µL, 1 mol/L), and the mixture was subjected to slurrying. The solid was separated out by centrifugation and dried *in vacuo* to obtain the product. The product was identified by X-ray powder diffraction as malate in amorphous form.

### Example 20

An ethyl acetate/heptane solution (V/V, 9:1, 0.25 mL) containing the compound of formula (I) (about 10 mg) was mixed with a solution of hippuric acid in ethanol (60 µL, 0.5 mol/L), and the mixture was subjected to slurrying. The solid was separated out by centrifugation and dried *in vacuo* to obtain the product. The product was identified by X-ray powder diffraction as hippurate in amorphous form.

### Test Examples:

### Biological Evaluation

### Test Example 1. Inhibitory Effect of Compounds Disclosed Herein on ATR Enzyme

The following method was used to determine the inhibitory effect of the compounds disclosed herein on ATR enzyme. The experimental method was briefly described as follows:
I. Experimental materials and instruments
   1. ATR enzyme (Eurofins Pharma Discovery Services, 14-953-M)
   2. GST-tag P53 protein (Eurofins Pharma Discovery Services, 14-952-M)
   3. 384-well plate (Thermo Scientific, 267462)
   4. U-shaped bottom 96-well plate (Corning, 3795)
   5. MAb Anti-phospho p53-Eu cryptate (Cisbio, 61P08KAE)
   6. MAb Anti GST-d2 (Cisbio, 61GSTDLF)
   7. ATP solution (Promega, V916B)
   8. EDTA (Thermo Scientific, AM9260G)
   9. HEPES (Gibco, 15630-080)
   10. Microplate reader (BMG, PHERAsta)
II. Experimental procedures
   1 nM ATR enzyme, 50 nM P53 protein, 7.435 µM ATP and small molecule compounds of different concentrations (serially 3-fold diluted from 1 µM to the 11^{th} concentration) were mixed and incubated at room temperature for 2 h. A terminating buffer (12.5 mM HEPES, 250 mM EDTA) was added. The mixture was well mixed before 0.42 ng/well of mAb anti-phospho p53-Eu cryptate and 25 ng/well of mAb anti GST-d2 were added. The mixture was incubated overnight at room temperature, and the fluorescence signals at 620 nm and 665 nm were detected using a PHERAstar system. Data were processed using GraphPad software.
III. Experimental data

The inhibitory activity of the compounds disclosed herein against ATR enzyme can be determined by the above assay, and the IC₅₀ values obtained are shown in Table 12.

**Table 12. IC₅₀ for ATR enzyme inhibition by compounds disclosed herein**

| Example No. | IC₅₀/nM | Max Inhibition (%) |
|---|---|---|
| 1 | 3 | 100 |

| | | |
|---|---|---|
| Conclusion: the compounds disclosed herein have good inhibitory activity against ATR enzyme. | | |

### Test Example 2. Cell Proliferation Assay

The following method evaluates the inhibitory effect of the compounds disclosed herein on the proliferation ofLoVo cells via IC₅₀ by measuring the intracellular ATP content. The experimental method was briefly described as follows:
I. Experimental materials and instruments
   1. LoVo, human colon cancer cells (Cobioer, Nanjing, CBP60032)
   2. Fetal bovine serum (GIBCO,10091-148)
   3. F-12K Medium (Gibco, 21127030)
   4. CellTite-Glo reagent (Promega, G7573)
   5. 96-well cell culture plate (Corning, 3903)
   6. Pancreatin (Invitrogen, 25200-072)
   7. Microplate reader (BMG, PHERAsta)
   8. Cell counter (Countstar, Shanghai, IC1000)
II. Experimental procedures

LoVo cells were cultured in an F-12K culture medium containing 10% of FBS, and passaged twice or thrice a week in a passage ratio of 1:3 or 1:5. During passage, cells were digested by pancreatin, transferred to a centrifuge tube, and centrifuged for 3 min at 1200 rpm. The supernatant was discarded, and fresh culture medium was added to resuspend the cells. To a 96-well cell culture plate, 90 µL of the cell suspension was added at a density of 3.88×10⁴ cells/mL. To peripheral wells of the 96-well plate, only 100 µL of complete medium was added. The culture plate was incubated in an incubator for 24 h (37 °C, 5% CO₂).

The test samples were each diluted to 2 mM in DMSO and serially 3-fold diluted to the 10^{th} concentration. Blank and control wells were set. 5 µL of the serially diluted test compound solution was added to 95 µL of fresh medium. 10 µL of the medium containing the compound above was added to the plate. The culture plate was incubated in an incubator for 3 days (37 °C, 5% CO₂). 50 µL of CellTiter-Glo reagent was added into each well of the 96-well cell culture plate. The plate was left to stand for 5-10 min in the dark at room temperature. The chemiluminescence signals were read by a PHERAstar system, and the data were processed by GraphPad software.

### III. Experimental data

The inhibitory activity of the compounds disclosed herein against LoVo cell proliferation can be determined by the above assay, and the IC₅₀ values obtained are shown in Table 13.

**Table 13. IC₅₀ for LoVo cell proliferation inhibition by compounds disclosed herein**

| Example No. | IC₅₀/nM | Max Inhibition (%) |
|---|---|---|
| 1 | 43 | 93 |

| | | |
|---|---|---|
| Conclusion: the compounds disclosed herein have good inhibitory activity against ATR enzyme. | | |

### Pharmacokinetic Evaluation

Test Example 3. Pharmacokinetic Study of Compounds Disclosed Herein

### 1. Abstract

The drug concentrations in the plasma of the test animals (rats) at different time points after intragastric administration of the compound of Example 1 were determined by an LC/MS/MS method. The pharmacokinetic behavior in rats of the compound disclosed herein was studied and its pharmacokinetic profile was evaluated.

### 2. Test protocol

### 2.1. Test drug

The compound of Example 1.

### 2.2. Test animals

12 healthy adult SD rats (half male and half female; purchased from Vital River) were evenly divided into 3 groups of 4.

### 2.3. Drug preparation

A certain amount of the compound was added to a mixed solvent containing 5% of DMSO, 5% of Tween 80 and 90% of normal saline to obtain a colorless and clear solution.

### 2.4. Administration

SD rats were intragastrically administered with the compound after fasting overnight, at a dose of 2 mg/kg and a volume of 10.0 mL/kg.

### 3. Procedures

Rats were intragastrically administered with the compound of Examples 1. 0.2 mL of blood was collected from the orbit pre-dose and at 0.25, 0.5, 1.0, 2.0, 4.0, 6.0, 8.0, 11.0 and 24.0 h post-dose. The blood samples were transferred to EDTA-K2kk anticoagulation tubes, centrifuged at 4 °C and 11000 rpm for 5 min to separate out plasma. The plasma samples were stored at -20 °C. The rats were fed 2 h after administration.

The plasma concentration of the compound in rats after intragastric administration was determined: 25 µL of rat plasma at each time point post-dose was mixed with 50 µL of internal standard and 175 µL of acetonitrile; the mixture was vortexed for 5 min and centrifuged for 10 min at 4000 rpm. 1 µL of supernatant was taken for LC/MS/MS analysis.

### 4. Pharmacokinetic parameters

**Table 14. The pharmacokinetic parameters of the compound disclosed herein**

| Example No. | Pharmacokinetic experiment (2 mg/kg) | | | | | |
|---|---|---|---|---|---|---|
| | Plasma concentration | Area under curve | Half-life | Residence time | Clearance | Apparent volume of distribution |
| | Cmax (ng /mL) | AUC (ng /mL*h) | T 1/2 (h) | MRT(h) | CL/F (ml/min/kg) | Vz/F (ml/kg) |
| 1 | 1112±394 | 3203±2747 | 2.62±2.48 | 3.08±2.31 | 17.7±12.4 | 2058±1222 |

| | | | | | | |
|---|---|---|---|---|---|---|
| Conclusion: The compound disclosed herein demonstrates good absorption profile and significant pharmacokinetic superiority. | | | | | | |

## Claims

1. A pharmaceutically acceptable salt of a compound of formula (I), wherein the pharmaceutically acceptable salt is selected from the group consisting of hydrochloride, sulfate, hydrobromide, mesylate, *p*-toluenesulfonate, maleate, phosphate, formate, acetate, succinate, fumarate, citrate, malate, hippurate and oxalate, and the pharmaceutically acceptable salt is preferably mesylate, maleate or oxalate,

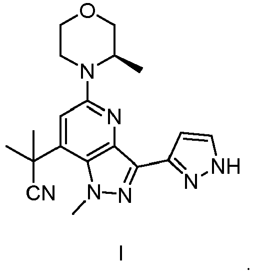

2. A crystalline form of hydrochloride of a compound of formula (I), wherein the crystalline form is:
a crystalline form a of hydrochloride having an X-ray powder diffraction pattern with characteristic peaks at 2θ angles of 6.0, 8.3, 12.1, 14.3, 14.9, 16.7 and 26.7, wherein preferably, the crystalline form a of hydrochloride has an X-ray powder diffraction pattern shown in FIG. 1;
a crystalline form b of hydrochloride having an X-ray powder diffraction pattern with characteristic peaks at 2θ angles of 6.0, 12.1, 18.2, 23.6 and 24.4, wherein preferably, the crystalline form b of hydrochloride has an X-ray powder diffraction pattern shown in FIG. 2.

3. A crystalline form α of sulfate of a compound of formula (I), having an X-ray powder diffraction pattern with characteristic peaks at 2θ angles of 5.8, 7.6, 13.7, 15.4 and 20.4, wherein preferably, the crystalline form α of sulfate has an X-ray powder diffraction pattern shown in FIG. 3.

4. A crystalline form of hydrobromide of a compound of formula (I), wherein the crystalline form is:
a crystalline form I of hydrobromide having an X-ray powder diffraction pattern with characteristic peaks at 2θ angles of 6.0, 8.1, 14.7, 25.9 and 27.0, wherein preferably, the crystalline form I of hydrobromide has an X-ray powder diffraction pattern shown in FIG. 4;
a crystalline form II of hydrobromide having an X-ray powder diffraction pattern with characteristic peaks at 2θ angles of 9.3, 11.6, 13.0, 16.8, 18.7 and 24.6, wherein preferably, the crystalline form II of hydrobromide has an X-ray powder diffraction pattern shown in FIG. 5.

5. A crystalline form of mesylate of a compound of formula (I), wherein the crystalline form is:
a crystalline form α of mesylate having an X-ray powder diffraction pattern with characteristic peaks at 2θ angles of 10.0, 16.8, 17.8, 18.4 and 20.6; or
a crystalline form β of mesylate having an X-ray powder diffraction pattern with characteristic peaks at 2θ angles of 5.9, 8.4, 14.5, 16.8, 19.8 and 26.0.

6. The crystalline form according to claim 5, being the crystalline form α of mesylate having an X-ray powder diffraction pattern with characteristic peaks at 2θ angles of 7.7, 10.0, 12.9, 13.8, 14.3, 15.1, 16.8, 17.8, 18.4, 20.3, 20.6, 21.9, 23.1, 24.2, 25.3, 26.1, 26.7, 28.3, 29.0, 30.7, 35.0 and 43.1, wherein preferably, the crystalline form α of mesylate has an X-ray powder diffraction pattern shown in FIG. 6.

7. A crystalline form I of maleate of a compound of formula (I), having an X-ray powder diffraction pattern with characteristic peaks at 2θ angles of 10.1, 17.1, 18.0, 19.0 and 24.3, wherein preferably, the crystalline form I of maleate has an X-ray powder diffraction pattern with characteristic peaks at 2θ angles of 7.2, 9.4, 10.1, 12.8, 13.2, 14.2, 14.8, 15.7, 17.1, 18.0, 19.0, 22.0, 23.4, 24.3, 25.2, 27.5 and 29.1; more preferably, the crystalline form I of maleate has an X-ray powder diffraction pattern shown in FIG. 8.

8. A crystalline form a of p-toluenesulfonate of a compound of formula (I), having an X-ray powder diffraction pattern with characteristic peaks at 2θ angles of 6.5, 8.6, 12.0, 14.5, 21.2 and 22.2, wherein preferably, the crystalline form a of *p*-toluenesulfonate has an X-ray powder diffraction pattern shown in FIG. 9.

9. A crystalline form a of oxalate of a compound of formula (I), having an X-ray powder diffraction pattern with characteristic peaks at 2θ angles of 5.5, 9.1, 11.0, 13.0, 16.5 and 20.2, wherein preferably, the crystalline form a of oxalate has an X-ray powder diffraction pattern with characteristic peaks at 2θ angles of 5.5, 9.1, 11.0, 13.0, 15.5, 16.5, 20.2, 22.0, 22.5, 23.1, 24.9, 26.2, 27.8 and 30.8; more preferably, the crystalline form a of oxalate has an X-ray powder diffraction pattern shown in FIG. 10.

10. The crystalline form of the salt of the compound of formula (I) according to any one of claims 2-9, wherein the 2θ angles have a margin of error of ±0.2.

11. A method for preparing the crystalline form α of mesylate of the compound of formula (I) according to claims 5-6 or claim 10, comprising: mixing a solution comprising the compound of formula (I) and methyl *tert*-butyl ether with methanesulfonic acid, slurrying and crystallizing.

12. A method for preparing the crystalline form I of maleate of the compound of formula (I) according to claim 7 or 10, comprising: mixing a solution comprising the compound of formula (I) and a solvent with maleic acid, slurrying and crystallizing, wherein the solvent is selected from the group consisting of methyl *tert*-butyl ether and ethyl acetate/heptane.

13. A method for preparing the crystalline form a of oxalate of the compound of formula (I) according to claim 9 or 10, comprising: mixing a solution comprising the compound of formula (I) and a solvent with oxalic acid, slurrying and crystallizing, wherein the solvent is selected from the group consisting of methyl *tert*-butyl ether and ethyl acetate/heptane.

14. A pharmaceutical composition, comprising the pharmaceutically acceptable salt of the compound of formula (I) according to claim 1 or the crystalline form of the pharmaceutically acceptable salt of the compound of formula (I) according to any one of claims 2-10, and one or more pharmaceutically acceptable carriers or excipients.

15. A method for preparing a pharmaceutical composition, comprising the step of: mixing the pharmaceutically acceptable salt of the compound of formula (I) according to claim 1 or the crystalline form of the pharmaceutically acceptable salt of the compound of formula (I) according to any one of claims 2-10 with one or more pharmaceutically acceptable carriers or excipients.

16. Use of the salt of the compound of formula (I) according to claim 1, the crystalline form of the pharmaceutically acceptable salt of the compound of formula (I) according to any one of claims 2-10 or the pharmaceutical composition according to claim 14 in preparing a medicament for inhibiting ATR kinase.

17. Use of the salt of the compound of formula (I) according to claim 1, the crystalline form of the pharmaceutically acceptable salt of the compound of formula (I) according to any one of claims 2-10 or the pharmaceutical composition according to claim 14 in preparing a medicament for treating a hyperproliferative disease.

18. Use of the salt of the compound of formula (I) according to claim 1, the crystalline form of the pharmaceutically acceptable salt of the compound of formula (I) according to any one of claims 2-10 or the pharmaceutical composition according to claim 14 in preparing a medicament for treating a tumor disease.
